# EUROPEAN PATENT APPLICATION

(11) **EP 4 628 485 A1**
(43) Date of publication of application: **08.10.2025**
(21) Application number: 22871158.6
(22) Date of filing: 20.12.2022
(51) Int. Cl.: C07D 317/36, B01J 31/02

(54) **USE OF CATALYST IN REACTION OF CATALYZING EPOXY COMPOUND AND CARBON DIOXIDE**

(30) Priority: 30.11.2022 CN 202211519088
(71) Applicant: Shenzhen Capchem Technology Co., Ltd, Shenzhen, Guangdong 518118 (CN)
(72) Inventor: KANG, Yuanyuan, Shenzhen, Guangdong 518118 (CN); CHEN, Xiaowei, Shenzhen, Guangdong 518118 (CN); TANG, Xiwu, Shenzhen, Guangdong 518118 (CN); ZHENG, Zhongtian, Shenzhen, Guangdong 518118 (CN)
(74) Representative: Predazzi, Valentina
(86) International application number: PCT/CN2022/140504
(87) International publication number: WO 2024/113437

(57) **Abstract**

The present disclosure provides use of a catalyst for catalyzing a reaction of an epoxide compound and carbon dioxide, in which the catalyst includes at least one compound of formula (1); where R is selected from an alkyl group, an alkenyl group, a halohydrocarbyl group, an aromatic hydrocarbyl group, an imidazolyl group, or a heteroaromatic hydrocarbyl group; X is selected from halogens; B is selected from nitrogen, or phosphorus; A is selected from formula (a), or formula (b).

## Description

### CROSS-REFERENCE TO RELATED APPLICATION

This application claims priority to Chinese Patent Application No. 202211519088.9, filed with the China National Intellectual Property Administration on November 30, 2022, the entire content of which is incorporated herein by reference.

### FIELD

The present disclosure relates to the technical field of chemical synthesis, and specifically relates to use of a catalyst for catalyzing a reaction of an epoxide compound and carbon dioxide.

### BACKGROUND

Carbon dioxide is a common and basic greenhouse gas, and is also an abundant C1 resource in nature. Reasonable utilization of carbon dioxide to obtain high-value chemical products is not only the manifestation of green chemistry, but also can alleviate the increasingly serious greenhouse effect caused by the continuous increase of carbon dioxide. Cyclic carbonate, as a solvent with good properties and an intermediate for fine chemical industry, is expected to become a new organic chemical foundation. Cyclic carbonate, as one of the compounds for many uses, is widely used for example as an organic solvent, a synthetic fiber processing agent, a pharmaceutical raw material, a cosmetic additive, and an electrolyte solvent for lithium batteries, and is mainly obtained from synthesis of carbon dioxide.

Addition reaction of carbon dioxide and an epoxide has advantages in industrial production. Existing catalysts on the market for the addition reaction are commonly binary catalysts composed of Lewis acid metal and Lewis base. These catalysts have defects, such as low catalytic activity, poor stability, harsh reaction condition requirement, toxicity, various by-products, and difficulty in product purification. Meanwhile, this type of reaction needs high temperature and high pressure environment, and has high device requirements. Therefore, a method of catalytic conversion of carbon dioxide with a high efficiency and under mild conditions to realize the effective utilization of carbon dioxide has attracted much attention.

Therefore, it is of great economic and environmental significance to develop catalysts and methods capable of catalyzing an addition reaction of carbon dioxide and the epoxide under environmentally friendly and mild conditions.

### SUMMARY

An object of the present disclosure is to provide use of a catalyst having a symmetrical structure for catalyzing a reaction of an epoxide compound and carbon dioxide.

In a first aspect, there is provided use of a catalyst for catalyzing an addition reaction of an epoxide compound and carbon dioxide, and the catalyst includes at least one compound of formula (1): 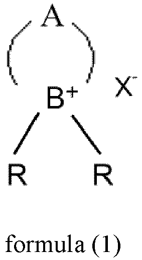 where R is selected from an alkyl group, an alkenyl group, a halohydrocarbyl group, an aromatic hydrocarbyl group, an imidazolyl group, or a heteroaromatic hydrocarbyl group; X is selected from halogens; B is selected from nitrogen, or phosphorus; A is selected from any of the following formulae with a sign * representing a bonding position:

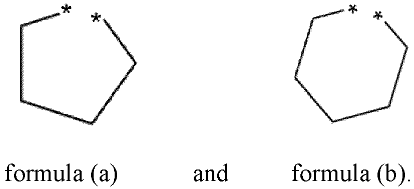

In some embodiments, R is selected from the alkyl group, the alkenyl group, or the halohydrocarbyl group, and has 1 to 15, preferably 1 to 10, carbon atoms.

In some embodiments, X is selected from F, Cl, Br, or I, preferably from Br, or Cl.

In some embodiments, the compound of formula (1) includes at least one of:

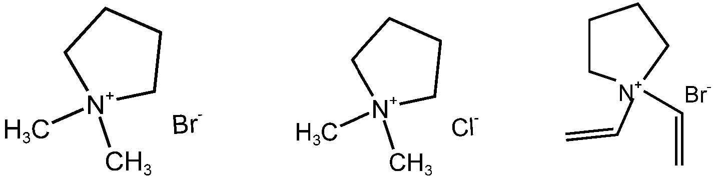

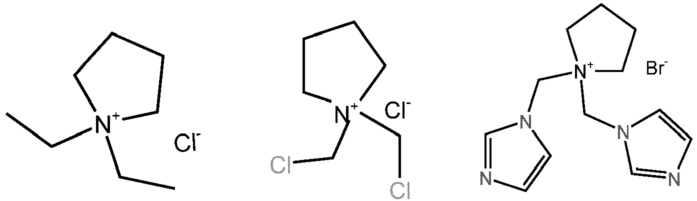

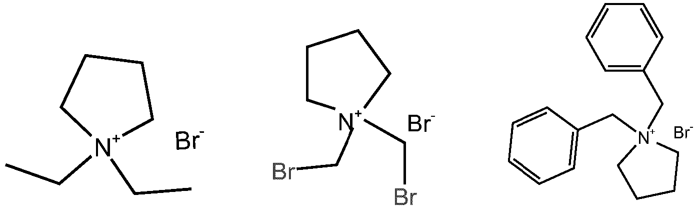

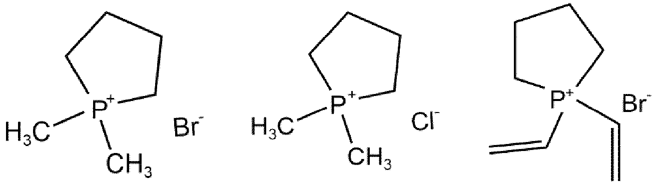

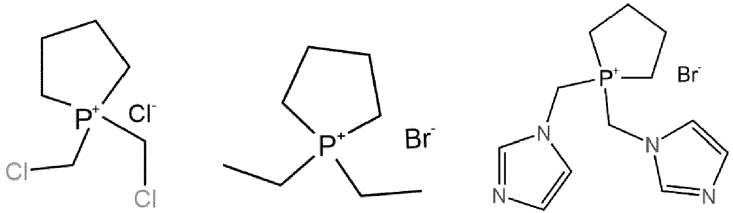

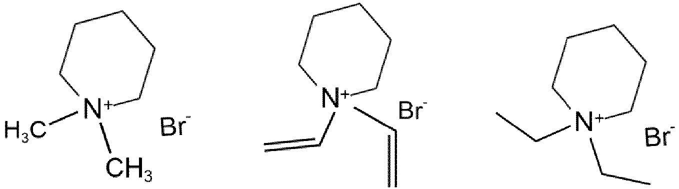

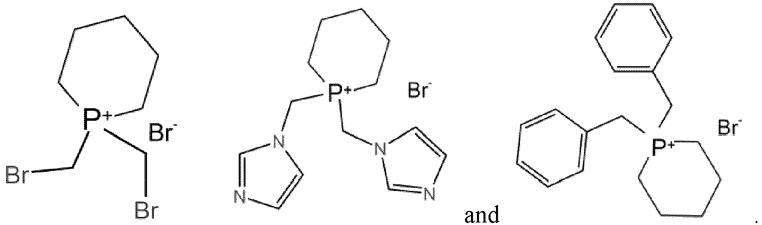

In some embodiments, the catalyst further includes at least one compound of formula (2): 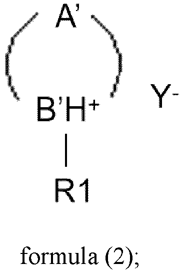 where R1 is selected from an alkyl group, an alkenyl group, a halohydrocarbyl group, an aromatic hydrocarbyl group, an imidazolyl group, or a heteroaromatic hydrocarbyl group; Y is selected from halogens; B' is selected from nitrogen, or phosphorus; A' is selected from any of the following formulae with a sign * representing a bonding position:

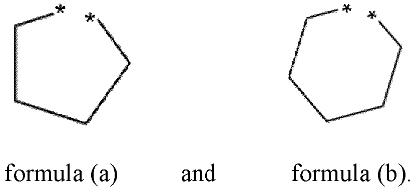

In some embodiments, R1 is selected from the alkyl group, the alkenyl group, or the halohydrocarbyl group, and has 1 to 15, preferably 1 to 10, carbon atoms; and/or, Y is selected from F, Cl, Br, or I, preferably from Br, or Cl.

In some embodiments, the compound of formula (2) includes at least one of:

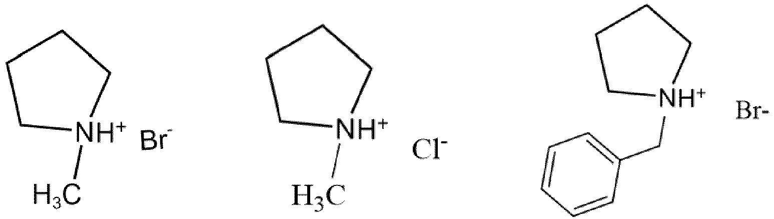

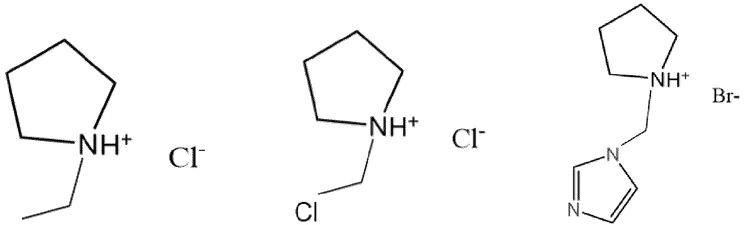

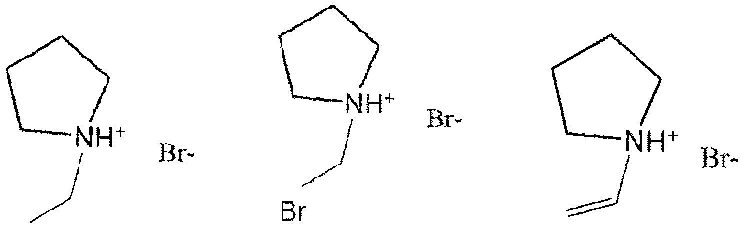

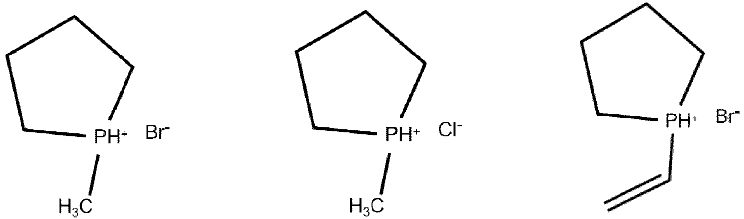

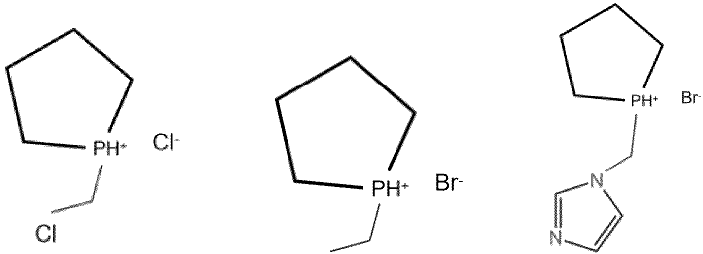

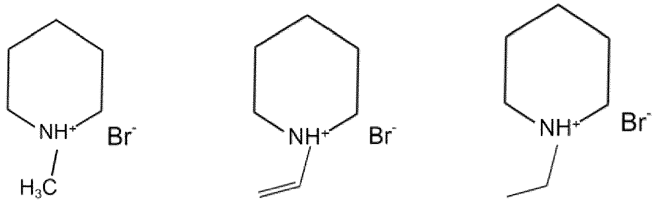

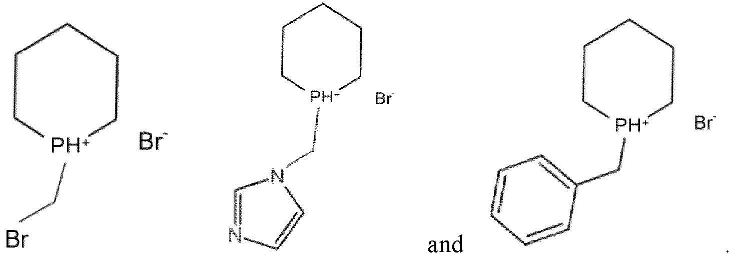

In some embodiments, a molar ratio of the compound of formula (1) to the compound of formula (2) is in a range of 100: (1 to 12).

In some embodiments, the epoxide compound is selected from at least one compound of formula (3): 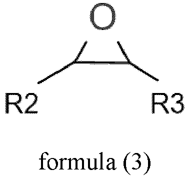 where when R2=H, R3 is selected from H, CH₃, CH₂Cl, C₂H₃, C₄H₉O, C₄H₉, C₆H₅, or C₈H₇O; when R2≠H, the epoxide compound is cyclohexene oxide.

In some embodiments, a molar ratio of the catalyst to the epoxide compound is in a range of (0.5 × 10⁻³ to 1.5 × 10⁻²):1.

In some embodiments, the reaction is performed at a temperature of 100 to 200 °C, and/or under a pressure of 1 to 5 MPa.

The present disclosure further provides in embodiments, a catalyst including at least one compound of formula (1) and at least one compound of formula (2): 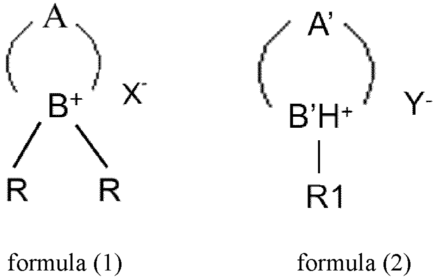 where R is selected from an alkyl group, an alkenyl group, a halohydrocarbyl group, an aromatic hydrocarbyl group, an imidazolyl group, or a heteroaromatic hydrocarbyl group; X is selected from halogens; B is selected from nitrogen, or phosphorus; and A is selected from any of the following formulae with a sign * representing a bonding position:

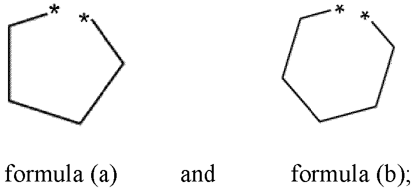

R1 is selected from an alkyl group, an alkenyl group, a halohydrocarbyl group, an aromatic hydrocarbyl group, an imidazolyl group, or a heteroaromatic hydrocarbyl group; Y is selected from halogens; B' is selected from nitrogen, or phosphorus; and A' is selected from any of the following formulae with a sign * representing a bonding position:

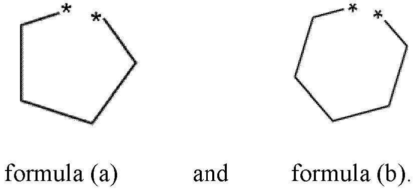

In some embodiments, R is selected from the alkyl group, the alkenyl group, or the halohydrocarbyl group, and has 1 to 15, preferably 1 to 10, carbon atoms; and/or, X is selected from F, Cl, Br, or I, preferably from Br, or Cl.

In some embodiments, the compound of formula (1) includes at least one of:

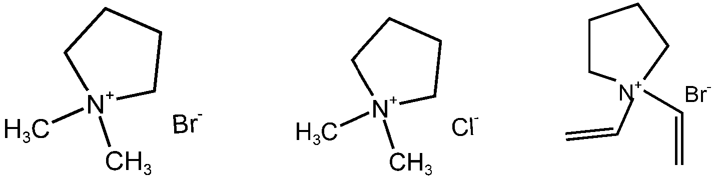

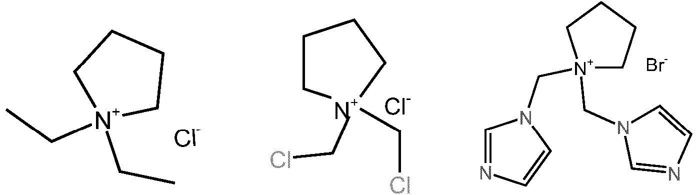

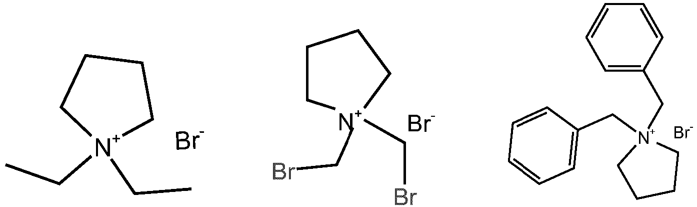

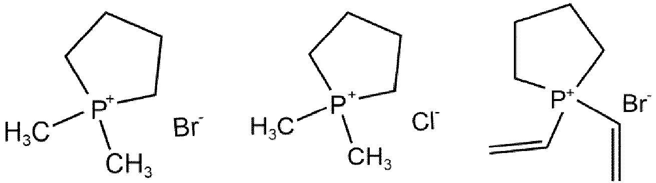

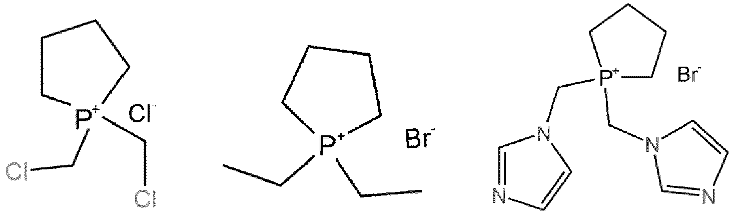

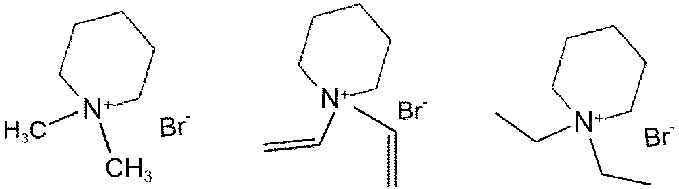

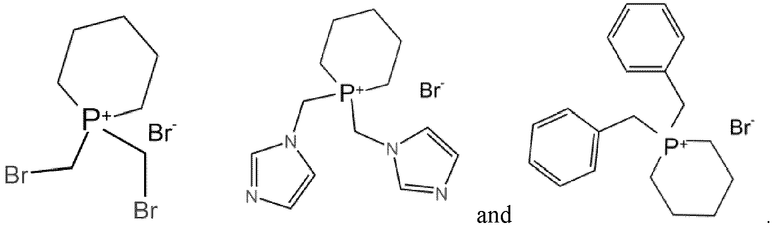

In some embodiments, the compound of formula (2) includes at least one of:

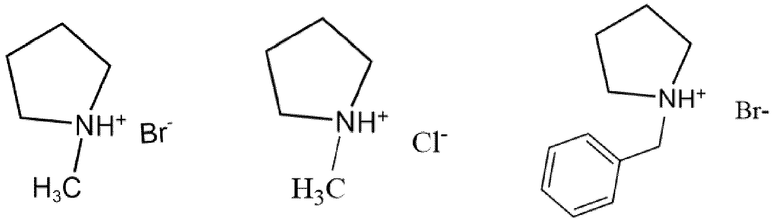

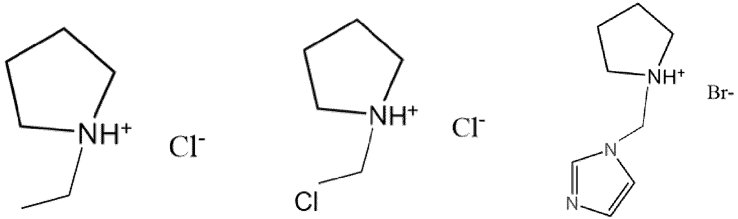

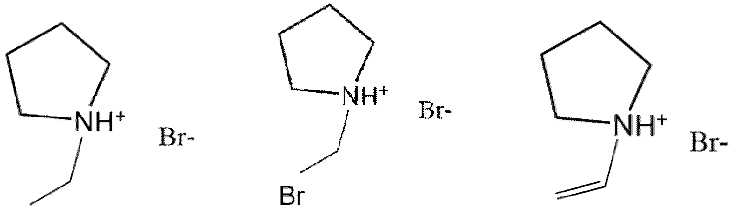

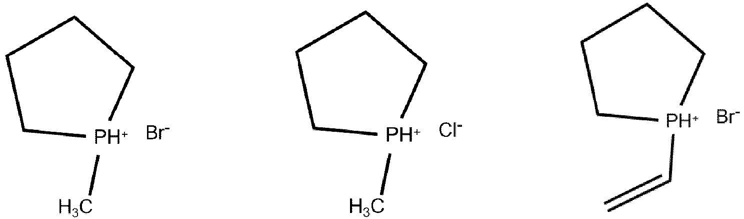

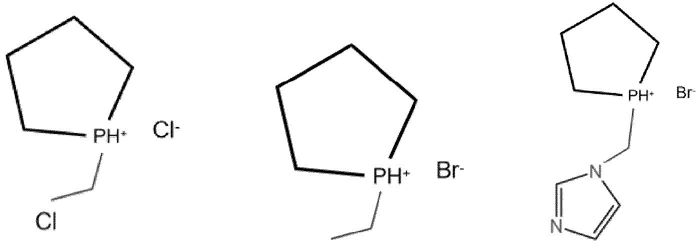

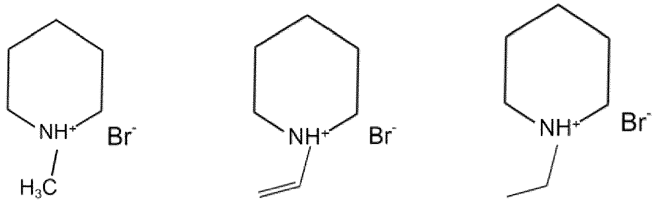

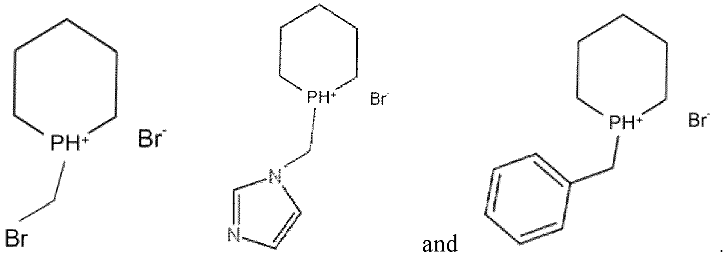

### BRIEF DESCRIPTION OF THE DRAWINGS

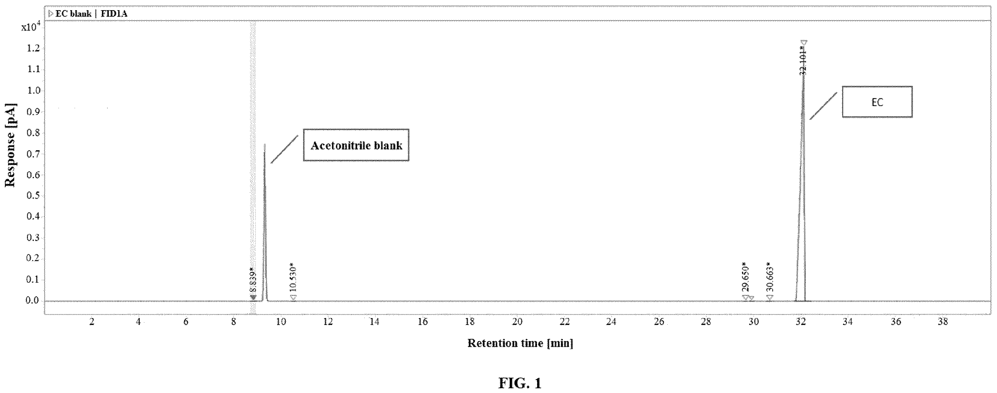

FIG. 1 is a gas chromatogram of a product catalyzed by a catalyst of the present disclosure.

### DETAILED DESCRIPTION

In the following embodiments of the present disclosure, if a condition for an experimental method or device is not specified, it is usually in accordance with conventional conditions, or conditions recommended by manufacturers. Various common chemicals used in the embodiments are commercially available products.

Unless defined otherwise, all technical and scientific terms used herein have the same meaning as commonly understood by those skilled in the art. Terms used in the specification of the present disclosure are only for the purpose of describing specific embodiments, and are not intended to limit the present disclosure.

Terms "including", "having", and any variations thereof of the present disclosure are intended to cover non-exclusive inclusions. For example, a process, a method, an apparatus, a product, or a device that includes a series of steps is not limited to the listed steps or modules only, but may further include steps that are not listed, or may include other steps inherent to the process, method, product or device.

In the present disclosure, the term "a plurality of" refers to two or more, and term "and/or" describes a relationship among associated objects, indicating that there may be three relationships. For example, A and/or B, may include three cases of A existing alone, A and B existing at the same time, and B existing alone. The character "/" generally indicates that the associated objects is in a relationship of being connected by "or".

This embodiment provides a use of a catalyst for catalyzing a reaction of an epoxide compound and carbon dioxide. The catalyst includes at least one compound of formula (1): 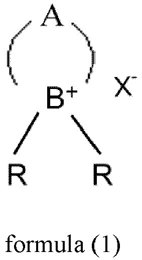 where R is selected from an alkyl group, an alkenyl group, a halohydrocarbyl group, an aromatic hydrocarbyl group, an imidazolyl group, or a heteroaromatic hydrocarbyl group; X is selected from halogens; B is selected from nitrogen, or phosphorus; A is selected from any of the following formulae with a sign * representing a bonding position:

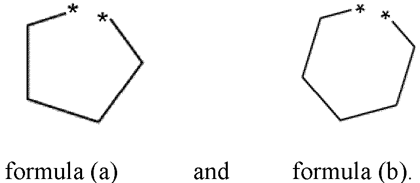

The compound of formula (1) is a catalyst with a symmetrical structure, and has a single type of reaction sites. It can be used in catalyzing the reaction of carbon dioxide and the epoxide compound to prepare a cyclic carbonate, and side reactions can be effectively reduced, thus resulting in a high final product purity and few impurity species.

In some embodiments, the compound of formula (1) includes, but is not limited to:

| | | |
|---|---|---|
| compound 1 | compound 2 | compound 3 |
| 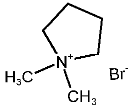 | 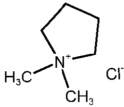 | 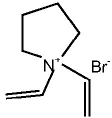 |
| compound 4 | compound 5 | compound 6 |
| 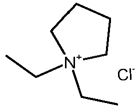 | 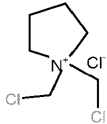 | 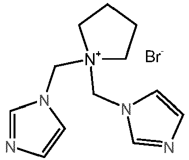 |
| compound 7 | compound 8 | compound 9 |
| 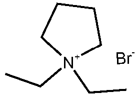 | 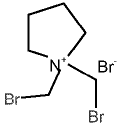 | 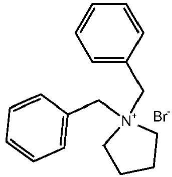 |
| compound 10 | compound 11 | compound 12 |
| 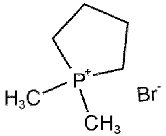 | 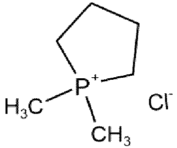 | 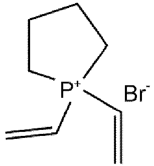 |
| compound 13 | compound 14 | compound 15 |
| 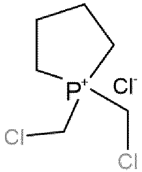 | 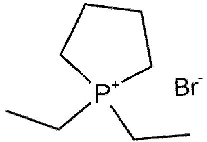 | 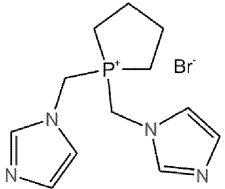 |
| compound 16 | compound 17 | compound 18 |
| 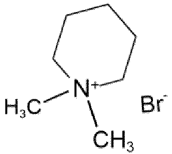 | 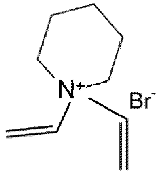 | 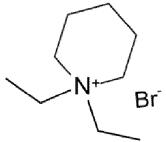 |
| compound 19 | compound 20 | compound 21 |
| 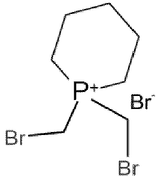 | 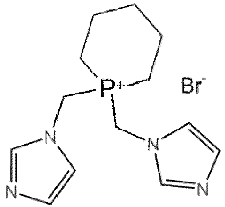 | 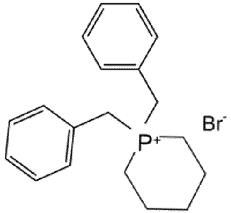 |

In another embodiment of the present disclosure, the catalyst further includes at least one compound of formula (2): 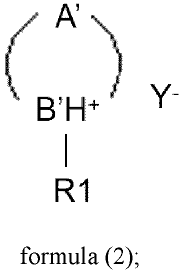 where R1 is selected from an alkyl group, an alkenyl group, a halohydrocarbyl group, an aromatic hydrocarbyl group, an imidazolyl group, or a heteroaromatic hydrocarbyl group; Y is selected from halogens; B' is selected from nitrogen, or phosphorus; A' is selected from any of the following formulae with a sign * representing a bonding position:

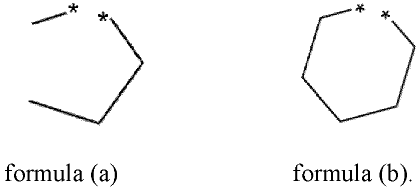

After a lot of researches, the inventors of the present disclosure found that a catalytic mechanism of the compound of formula (2) is similar to that of the compound of formula (1), but it has a stronger adsorption to the oxygen free radicals formed from the epoxides (in which the ring of the epoxide is opened and is further reacted with halogen). When the compound of formula (1) is compounded with a small amount of the compound of formula (2), the above-mentioned oxygen free radicals can be transferred, and the compound of formula (1) can be released, thus reducing decomposition caused by nucleophilic adsorption. When the compound of formula (1) and the compound of formula (2) are used as a composite catalyst in a proper proportion, the stability of the catalyst used for cycles can be improved, and a good catalytic activity can still be maintained after a plurality of cycles.

In some embodiments, a molar ratio of the compound of formula (1) to the compound of formula (2) in the composite catalyst is in a range of 100: (1 to 12). If the content of the compound of formula (2) is too small, the effect of increasing the stability will not be realized. If the content is too high, an amount of impurities in the product will be increased.

In some embodiment, the compound of formula (2) includes, but is not limit to:

| | | |
|---|---|---|
| compound 22 | compound 23 | compound 24 |
| 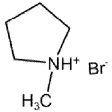 | 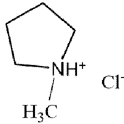 | 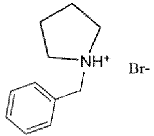 |
| compound 25 | compound 26 | compound 27 |
| 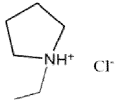 | 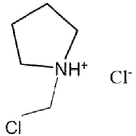 | 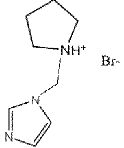 |
| compound 28 | compound 29 | compound 30 |
| 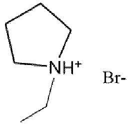 | 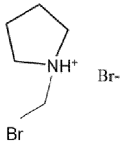 | 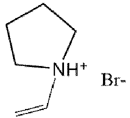 |
| compound 31 | compound 32 | compound 33 |
| 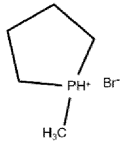 | 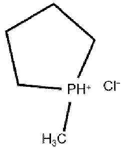 | 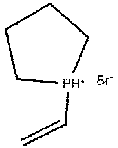 |
| compound 34 | compound 35 | compound 36 |
| 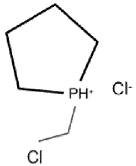 | 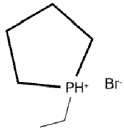 | 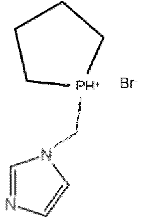 |
| compound 37 | compound 38 | compound 39 |
| 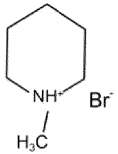 | 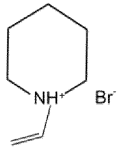 | 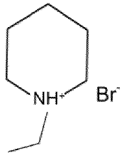 |
| compound 40 | compound 41 | compound 42 |
| 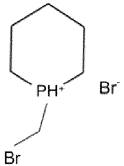 | 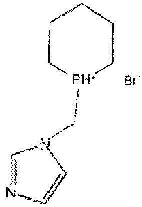 | 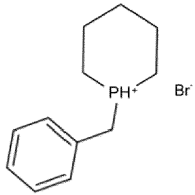 |

In some embodiments, the composite catalyst includes the compound 1 and the compound 22, or the compound 2 and the compound 23, or the compound 9 and the compound 24.

The general reaction formula of the compound of formula (1) in the present disclosure is as follows.

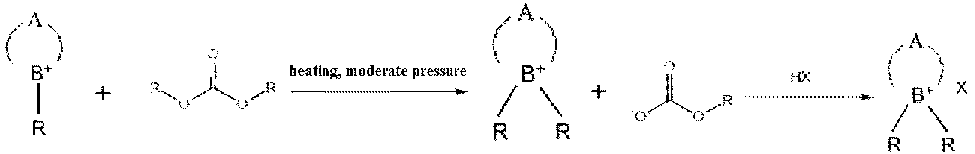

The general reaction formula of the compound of formula (2) in the present disclosure is the same as that of the compound of formula (1), except that a raw material 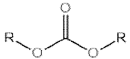 of the reaction is replaced by one material of a similar formula with one R determined as H.

With the formulae of the compounds of formula (1) and formula (2), preparation methods of the above-mentioned compounds can be prepared according to the common knowledge in the chemical synthesis field. For example, the compound 1 can be prepared as follows.

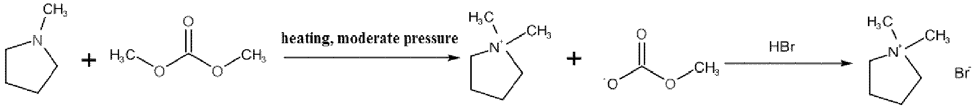

In some embodiments, the epoxide compound is selected from at least one compound of formula (3): 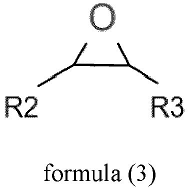 where when R2=H, R3 is selected from H (ethylene oxide), CH₃ (propylene oxide), CH₂Cl (epichlorohydrin), C₂H₃ (butadiene monoxide), C₄H₉O (2-propoxymethyloxirane), C₄H₉ (1,2-epoxyhexane), C₆H₅ (styrene oxide), or C₈H₇O (2-(phenoxymethyl)oxirane); when R2≠H, the epoxide compound is cyclohexene oxide.

In some embodiments, the general formula of the catalyst for catalyzing the reaction of the epoxide compound and carbon dioxide is as follows: 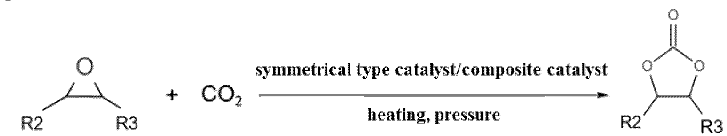 where when R2=H, R3 is selected from H (ethylene oxide), CH₃ (propylene oxide), CH₂Cl (epichlorohydrin), C₂H₃ (butadiene monoxide), C₄H₉O (2-propoxymethyloxirane), C₄H₉ (1,2-epoxyhexane), C₆H₅ (styrene oxide), or C₈H₇O (2-(phenoxymethyl)oxirane); when R2≠H, the epoxide compound is cyclohexene oxide.

In some embodiments, a molar ratio of the catalyst to the epoxide compound is in a range of (0.5 × 10⁻³ to 1.5 × 10⁻²):1. Preferably, the molar ratio of the catalyst to the epoxide compound is in a range of (0.5× 10⁻³ to 5× 10⁻³):1.

In some embodiments, a temperature of the reaction is in a range of 100 to 200°C. Preferably, the temperature of the reaction is in a range of 120 to 160°C.

In some embodiments, a pressure of the reaction is in a range of 1 to 5 MPa. Preferably, the pressure of the reaction is in a range of 1 to 3 MPa.

The present disclosure is further described with reference to the following examples.

### Example 1

In this example, a method for preparing a cyclic carbonate is provided, and a reaction formula of the method is as follows:

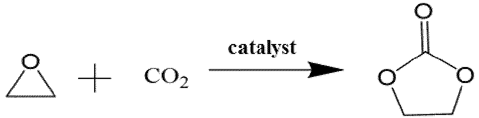

Experimental groups 1 to 10 and control groups 1 to 2 are provided. A preparation method for the experimental groups 1 to 10 includes the following operations (1) to (6).
(1) For each of experimental groups 1 to 10, a catalyst is added in a 100 mL stainless steel reactor, and a type and an amount of the catalyst is shown in Table 1.
(2) 44 g ethylene oxide (1 mol) is introduced into the stainless steel reactor.
(3) The reactor is sealed and filled with carbon dioxide at any appropriate pressure to make a pressure of the system to be about 1.0 MPa.
(4) The temperature is slowly increased by a temperature controller, and a final temperature is shown in Table 1.
(5) The pressure of carbon dioxide is controlled to be 1 to 5 Mpa, preferably 1 to 3 MPa, as shown in Table 1.
(6) After the reaction is the completed, the reactor is cooled to the room temperature, the product is discharged from the reactor. Remaining carbon dioxide is absorbed with saturated sodium carbonate solution. Liquid obtained after the reaction is decompressed and distilled to obtain the product cyclic carbonate.

For control groups 1 to 2, the preparation method for the control groups 1 to 2 is the same as that for the experiment group 1 (as shown in Table 1) except for the type of the catalyst. Control groups 1 and 2 use the following catalysts, Control 1 and Control 2, respectively.

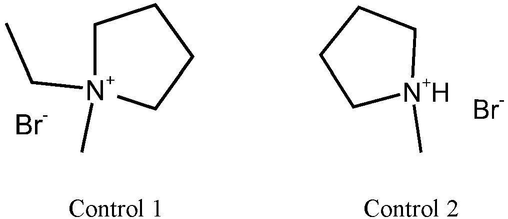

**Table 1**

| Group | Catalyst | Adding amount | Reaction time | Reaction pressure | Reaction temperature |
|---|---|---|---|---|---|
| Experimental group 1 | compound 1 | 1mmol | 2h | 2Mpa | 140°C |
| Experimental group 2 | compound 4 | 1mmol | 2h | 2Mpa | 120°C |
| Experimental group 3 | compound 1 | 0.5mmol | 2h | 2.5Mpa | 140°C |
| Experimental group 4 | compound 2 | 1mmol | 2h | 3Mpa | 140°C |
| Experimental group 5 | compound 6 | 1mmol | 2h | 1.5Mpa | 140°C |
| Experimental group 6 | compound 12 | 12mmol | 2h | 3.5Mpa | 130°C |
| Experimental group 7 | compound 15 | 1mmol | 2h | 2Mpa | 160°C |
| Experimental group 8 | compound 17 | 5mmol | 2h | 4.5Mpa | 175°C |
| Experimental group 9 | compound 20 | 15mmol | 2h | 2Mpa | 105°C |
| Experimental group 10 | compound 1+ compound 22 | 1mmol | 1.5h | 2Mpa | 140°C |
| Control group 1 | Control 1 | 1mmol | 2h | 2Mpa | 140°C |
| Control group 2 | Control 2 | 1mmol | 2h | 2Mpa | 140°C |

In Table 1, a molar ratio of the compound 1 to the compound 22 in the composite catalyst used in the experimental group 10 is 20:1.

Test results of the above experimental groups and control groups are shown in Table 2 below. Gas chromatographic data of a product of the experimental group 1 is shown in FIG. 1.

**Table 2**

| Group | Selectivity | Yield | Final product purity | Impurity species |
|---|---|---|---|---|
| Experimental group 1 | 98.0% | 99.1% | 99.95% | 5 |
| Experimental group 2 | 97.3% | 96.3% | 99.93% | 5 |
| Experimental group 3 | 98.1% | 97.4% | 99.94% | 5 |
| Experimental group 4 | 96.2% | 95.7% | 99.74% | 5 |
| Experimental group 5 | 95.2% | 95.0% | 99.38% | 6 |
| Experimental group 6 | 95.3% | 95.1% | 99.43% | 6 |
| Experimental group 7 | 95.0% | 94.5% | 99.30% | 6 |
| Experimental group 8 | 95.4% | 95.2% | 99.45% | 6 |
| Experimental group 9 | 95.1% | 94.8% | 99.36% | 6 |
| Experimental group 10 | 99.2% | 99.1% | 99.94% | 7 |
| Control group 1 | 96.6% | 94.5% | 99.00% | 9 |
| Control group 2 | 95.8% | 94.1% | 98.53% | 10 |

| | | | | |
|---|---|---|---|---|
| Note: the number of impurity species refers to the number of impurity peaks except for peaks of the cyclic carbonate and acetonitrile blank. | | | | |

The above results show that the preparation methods for the experimental groups 1 to 10 have advantages of good selectivity, yield, and final product purity, as well as few impurity species. Compared with the result of the experimental group 1, the selectivities, yields and final product purities of the control groups 1 and 2 are inferior, and more impurity species are obtained in control groups 1 and 2. It shows that the catalyst used in the preparation method of the present disclosure can effectively reduce the side reactions, improve the final product purity and reduce the impurity species.

### Example 2

In this example, a method for preparing a cyclic carbonate is provided, and a reaction formula of the method is as follows:

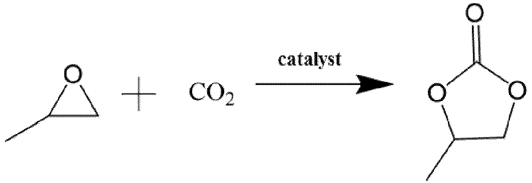

Experimental groups 11 to 17 and control groups 3 to 4 are provided. Preparation method for the experimental groups 11 to 17 includes the following operations (1) to (6).
(1) For each of the experimental groups 11 to 17, a catalyst is added in a 100 mL stainless steel reactor, and a type and an amount of the catalyst is shown in Table 3.
(2) 58.0 g methyl ethylene oxide (1 mol) is introduced into the stainless steel reactor.
(3) The reactor is sealed and filled with carbon dioxide at any appropriate pressure.
(4) The temperature is slowly increased by a temperature controller, and a final temperature is shown in Table 3.
(5) The pressure of carbon dioxide is controlled to a value as shown in Table 3.
(6) After the reaction is the completed, the reactor is cooled to the room temperature, the product is discharged from the reactor. Remaining carbon dioxide is absorbed with saturated sodium carbonate solution. Liquid obtained after the reaction is decompressed and distilled to obtain the product cyclic carbonate.

For control groups 3 and 4: as shown in Table 3, the preparation method for the control group 3 is the same as that for the experimental group 11 except for the type of the catalyst, and the control group 3 uses the catalyst Control 1. The preparation method in the control group 4 is the same as that for the experimental group 16 except for the type of the catalyst, and the control group 4 uses the catalyst Control 2.

**Table 3**

| Group | Catalyst | Adding amount | Reaction time | Reaction pressure | Reaction temperature |
|---|---|---|---|---|---|
| Experimental group 11 | compound 1 | 1mmol | 2h | 2Mpa | 140°C |
| Experimental group 12 | compound 1 | 1mmol | 2h | 2Mpa | 120°C |
| Experimental group 13 | compound 5 | 0.5mmol | 2h | 2.5Mpa | 140°C |
| Experimental group 14 | compound 2 | 1mmol | 2h | 3Mpa | 140°C |
| Experimental group 15 | compound 3 | 1mmol | 2h | 1.5Mpa | 140°C |
| Experimental group 16 | compound 1 | 1mmol | 2h | 2Mpa | 160°C |
| Experimental group 17 | compound 2+ compound 23 | 1mmol | 1.5h | 2Mpa | 140°C |
| Control group 3 | Control 1 | 1mmol | 2h | 2Mpa | 140°C |
| Control group 4 | Control 2 | 1mmol | 2h | 2Mpa | 160°C |

In Table 2, a molar ratio of the compound 2 to the compound 23 in the composite catalyst used in the experimental group 17 is 100:10.

Test results of selectivity, yield, final product purity and impurity quantity of the above experimental groups and control groups are shown in Table 4 below:

**Table 4**

| Group | Selectivity | Yield | Final product purity | Impurity species |
|---|---|---|---|---|
| Experimental group 11 | 96.8% | 96.7% | 99.85% | 5 |
| Experimental group 12 | 95.6% | 95.4% | 99.26% | 5 |
| Experimental group 13 | 92.7% | 91.0% | 99.63% | 5 |
| Experimental group 14 | 92.1% | 90.8% | 98.75% | 6 |
| Experimental group 15 | 91.0% | 88.9% | 99.07% | 6 |
| Experimental group 16 | 96.5% | 96.1% | 99.79% | 5 |
| Experimental group 17 | 92.4% | 91.1% | 99.01% | 7 |
| Control group 3 | 92.6% | 93.9% | 98.21% | 9 |
| Control group 4 | 91.6% | 93.4% | 98.03% | 10 |

| | | | | |
|---|---|---|---|---|
| Note: the number of impurity species refers to the number of impurity peaks except for peaks of the cyclic carbonate and acetonitrile blank. | | | | |

The above results show that the preparation methods for the experimental groups 11 to 17 have advantages of good selectivity, yield and final product purity, as well as few impurity species. Compared with the experimental group 11, the selectivity, yield and final product purity of the control group 3 are inferior, and more impurity species are obtained in control group 3. Compared with the experimental group 16, the selectivity, yield and final product purity of the control group 4 are inferior, and more impurity species are obtained in control group 4. It shows that the catalyst used in the preparation method of the present disclosure can effectively reduce the side reactions, improve the final product purity and reduce the impurity species.

### Example 3

In this example, a method for preparing a cyclic carbonate is provided, and a reaction formula of the preparation method is as follows:

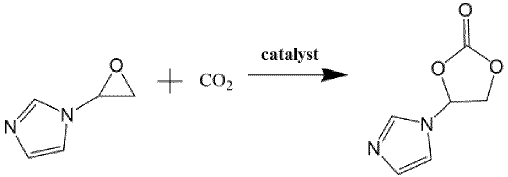

Experimental groups 18 to 24 and control groups 5 to 6 are provided. Preparation methods for the experimental groups 18 to 24 includes the following operations (1) to (6).
(1) An additive is added in a 100 mL stainless steel reactor at a specific content as shown in Table 5.
(2) 77 g 1,3-diazacyclopentadiene oxirane (0.5 mol) is introduced into the stainless steel reactor.
(3) The reactor is sealed and filled with carbon dioxide at any appropriate pressure.
(4) The temperature is slowly increased by a temperature controller, and a final temperature is shown in Table 5.
(5) The pressure of the carbon dioxide is controlled to a value as shown in Table 5.
(6) After the reaction is the completed, the reactor is cooled to the room temperature, the product is discharged from the reactor. Remaining carbon dioxide is absorbed with saturated sodium carbonate solution. Liquid obtained after the reaction is decompressed and distilled to obtain the product cyclic carbonate.

For control groups 5 and 6: as shown in Table 5, the preparation method for the control group 5 is the same as that for the experimental group 23 except for the type of the catalyst, and the control group 5 uses the catalyst Control 1. The preparation method in the control group 6 is the same as that for the experimental group 18 except for the type of the catalyst, and the control group 6 uses the catalyst Control 2.

**Table 5**

| Group | Catalyst | Adding amount | Reaction time | Reaction pressure | Reaction temperature |
|---|---|---|---|---|---|
| Experimental group 18 | compound 1 | 1mmol | 2h | 2Mpa | 140°C |
| Experimental group 19 | compound 7 | 1mmol | 2h | 2Mpa | 120°C |
| Experimental group 20 | compound 1 | 0.5mmol | 2h | 2.5Mpa | 140°C |
| Experimental group 21 | compound 2 | 1mmol | 2h | 3Mpa | 140°C |
| Experimental group 22 | compound 8 | 1mmol | 2h | 1.5Mpa | 140°C |
| Experimental group 23 | compound 1 | 1mmol | 2h | 2Mpa | 150°C |
| Experimental group 24 | compound 9+ compound 24 | 1mmol | 1.5h | 2Mpa | 140°C |
| Control group 5 | Control 1 | 1mmol | 2h | 2Mpa | 150°C |
| Control group 6 | Control 2 | 1mmol | 2h | 2Mpa | 140°C |

In Table 3, a molar ratio of the compound 9 to the compound 24 in the composite catalyst used in the experimental group 24 is 100:4.

Test results of the above experimental groups and control groups are shown in Table 6 below:

**Table 6**

| Group | Selectivity | Yield | Final product purity | Impurity species |
|---|---|---|---|---|
| Experimental group 18 | 93.4% | 90.7% | 99.65% | 7 |
| Experimental group 19 | 88.5% | 87.9% | 99.48% | 7 |
| Experimental group 20 | 90.8% | 88.3% | 99.73% | 7 |
| Experimental group 21 | 80.9% | 85.9% | 98.24% | 8 |
| Experimental group 22 | 76.8% | 83.7% | 97.91% | 8 |
| Experimental group 23 | 93.6% | 91.1% | 99.71% | 7 |
| Experimental group 24 | 96.5% | 95.1% | 99.94% | 7 |
| Control group 5 | 80.3% | 75.7% | 98.29% | 14 |
| Control group 6 | 66.8% | 38.6% | 98.01% | 14 |

| | | | | |
|---|---|---|---|---|
| Note: the number of impurity species refers to the number of impurity peaks except for peaks of the cyclic carbonate and acetonitrile blank. | | | | |

The above results show that the preparation methods for the experimental groups 18 to 24 have advantages of good selectivity, yield and final product purity, as well as few impurity species. Compared with the experimental group 18, the selectivity, yield and final product purity of the control group 6 are inferior, and more impurity species are obtained in control group 6. Compared with the experimental group 23, the selectivity, yield and final product purity of the control group 5 are inferior, and more impurity species are obtained in control group 5. It shows that the catalyst used in the preparation method of the present disclosure can effectively reduce the side reactions, improve the final product purity and reduce the impurity species.

### Example 4

In this example, a catalytic stability of a catalyst is analyzed.

Experimental groups 25 to 30 and control groups 7 to 8 are provided. The preparation methods for all these groups are the same as that for the experimental group 1 in Example 1 except that the catalysts used are different. The catalyst used in each group is shown in Table 7.

**Table 7**

| Group | Catalyst | Mole ratio | Adding amount | Reaction time | Reaction pressure | Reaction temperature |
|---|---|---|---|---|---|---|
| Experimental group 25 | compound 1 | -- | 1mmol | 2h | 2Mpa | 140°C |
| Experimental group 26 | compound 1+ compound 22 | compound 1: compound 22 =100: 5 | | | | |
| Experimental group 27 | compound 2+ compound 23 | compound 2: compound 23 =100:10 | | | | |
| Experimental group 28 | compound 9+ compound 24 | compound 9: compound 24 =100: 4 | | | | |
| Experimental group 29 | compound 9+ compound 24 | compound 9: compound 24 =100: 0.5 | | | | |
| Experimental group 30 | compound 9+ compound 24 | compound 9: compound 24 =100:13 | | | | |
| Control group 7 | Control 1 | -- | | | | |
| Control group 8 | Control 2 | -- | | | | |

After separating off newly formed cyclic carbonate, ethylene oxide and carbon dioxide are introduced into the reaction system for reaction repeatedly. The number of cycles of the catalyst and corresponding test results are shown in Table 8.

**Table 8**

| Group | Number of cycles | Selectivity | Yield | Final product purity | Impurity species |
|---|---|---|---|---|---|
| Experimental group 25 | 3 | 97.9% | 99.0% | 99.95% | 5 |
| | 4 | 98.0% | 98.9% | 99.94% | 5 |
| | 5 | 95.2% | 96.9% | 99.90% | 6 |
| Experimental group 26 | 3 | 99.8% | 99.5% | 99.99% | 7 |
| | 4 | 99.7% | 99.5% | 99.98% | 7 |
| | 5 | 99.8% | 99.4% | 99.99% | 7 |
| Experimental group 27 | 3 | 98.6% | 99.5% | 99.99% | 7 |
| | 4 | 98.6% | 99.3% | 99.99% | 7 |
| | 5 | 98.5% | 99.3% | 99.97% | 7 |
| Experimental group 28 | 3 | 98.8% | 98.6% | 99.99% | 7 |
| | 4 | 98.6% | 98.4% | 99.98% | 7 |
| | 5 | 98.7% | 98.3% | 99.96% | 8 |
| Experimental group 29 | 3 | 98.8% | 98.6% | 99.89% | 7 |
| | 4 | 98.6% | 98.4% | 99.88% | 7 |
| | 5 | 97.7% | 97.8% | 99.59% | 7 |
| Experimental group 30 | 3 | 98.8% | 98.6% | 99.99% | 7 |
| | 4 | 98.6% | 98.5% | 99.98% | 8 |
| | 5 | 98.6% | 98.4% | 99.98% | 8 |
| Control group 7 | 3 | 96.5% | 94.4% | 98.98% | 10 |
| | 4 | 96.3% | 94.2% | 98.89% | 11 |
| | 5 | 94.4% | 92.8% | 98.78% | 11 |
| Control group 8 | 3 | 95.2% | 94.0% | 98.11% | 11 |
| | 4 | 95.0% | 93.9% | 98.08% | 12 |
| | 5 | 94.6% | 93.7% | 98.01% | 13 |

| | | | | | |
|---|---|---|---|---|---|
| Note: the number of impurity species refers to the number of impurity peaks except for peaks of the cyclic carbonate and acetonitrile blank. | | | | | |

From the above results, it can be seen that compared with the catalysts used in the control groups, the catalysts of the present disclosure have higher catalytic activity and better catalytic stability after cycles.

It can be seen from the comparison between the experimental group 28 and the experimental groups 29 to 30 that the ratio of the compound of formula (1) to the compound of formula (2) in the composite catalyst may affect the catalytic effect of the composite catalyst. If the content of the compound of formula (2) in the composite catalyst is too low (the experimental group 29), the effect of increased stability may not be that good. If the content is too high (the experimental group 30), the impurity species may increase.

It can be seen from the comparison between the experimental group 25 and the experimental groups 26 to 28 that the catalytic stability of the composite catalyst composed of the compound of formula (1) and the compound of formula (2) is better than that of the single-component catalyst, i.e., the compound of formula (1). Further, the selectivity, yield and product purity achieved by the composite catalyst are substantially the same after the catalyst is used for 5 cycles, which shows that the catalytic effect of the composite catalyst is more stable.

The present disclosure provides a catalyst with a symmetrical structure in use for catalyzing a reaction of an epoxide compound and carbon dioxide, and the catalyst has the general structural formula (1). The catalyst can catalyze the addition reaction of carbon dioxide and the epoxide compound under environmentally friendly and mild conditions. Through researches, it is found that the compound of formula (1) is able to overcome the problems of low catalytic performance, long reaction time, strong toxicity and the like existed in the reaction process of the existing catalyst for catalyzing the reaction of carbon dioxide and the epoxide compound. In addition, the present catalyst has less complex reaction sites, so that it can effectively reduce the side reactions in catalyzing the addition reaction of carbon dioxide and the epoxide compound, thus improving the final product purity and reducing the impurity species. Further, it is found in the present disclosure that the catalytic mechanism of the compound of formula (2) is similar to that of the compound of formula (1), but it has a stronger adsorption to the oxygen free radicals formed from the epoxides (in which the ring of the epoxide is opened and is further reacted with halogen). When the compound of formula (1) is compounded with a small amount of the compound of formula (2), the above-mentioned oxygen free radicals can be transferred, and the compound of formula (1) can be released, thus reducing decomposition caused by nucleophilic adsorption. When the compound of formula (1) and the compound of formula (2) are used as the composite catalyst in a proper proportion, the stability of the catalyst used for cycles can be improved, and a good catalytic activity can still be maintained after a plurality of cycles.

To sum up, in the present disclosure, the compound of formula (1) is used as the catalyst to catalyze the reaction of carbon dioxide and the epoxide compound under environmentally friendly and mild conditions, and the obtained product has higher purity and fewer impurity species. Further, when the compound of formula (1) and the compound of formula (2) are used as the composite catalyst, the catalytic stability is significantly improved, and the good catalytic activity can be maintained after a plurality of cycles.

Technical features of the above-mentioned embodiments can be combined in any combination, and in order to make the description concise, not all the possible combinations of the technical features of the above-mentioned embodiments are described herein. However, as long as there is no contradiction between the technical features in the combination, the features can be combined and should be considered as being in the scope of the present description.

The embodiments described above are merely representative of several embodiments of the present disclosure and are described in detail, but are not to be construed as limiting the scope of the present disclosure. It is to be noted that those person skilled in the art could further make several variations and modifications without departing from the concept of the present disclosure, which are within the scope of the present disclosure. Accordingly, the protection scope of the present disclosure is as set forth in the appending claims.

## Claims

1. Use of a catalyst for catalyzing a reaction of an epoxide compound and carbon dioxide, wherein the catalyst comprises at least one compound of formula (1): 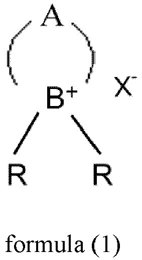 wherein R is selected from an alkyl group, an alkenyl group, a halohydrocarbyl group, an aromatic hydrocarbyl group, an imidazolyl group, or a heteroaromatic hydrocarbyl group; X is selected from halogens; B is selected from nitrogen, or phosphorus; A is selected from any of the following formulae with a sign * representing a bonding position:

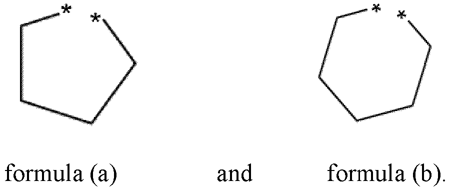

2. The use of claim 1, wherein R is selected from the alkyl group, the alkenyl group, or the halohydrocarbyl group, and has 1 to 15, preferably 1 to 10, carbon atoms; and/or, X is selected from F, Cl, Br, or I, preferably from Br, or Cl.

3. The use of claim 1, wherein the compound of formula (1) comprises at least one of:

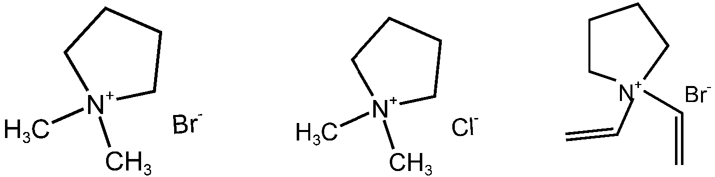

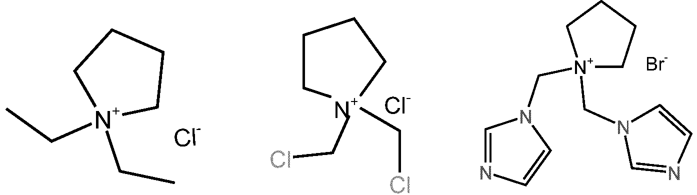

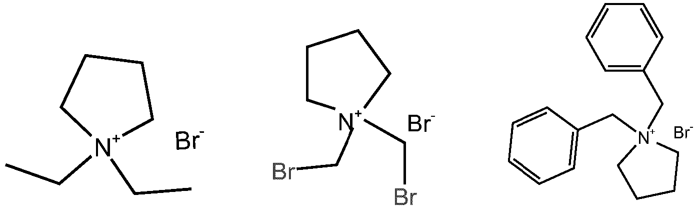

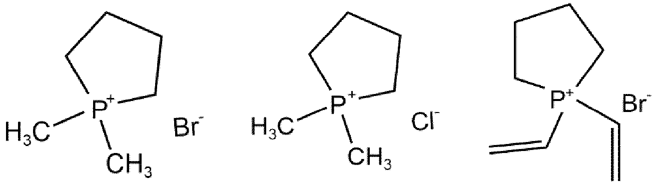

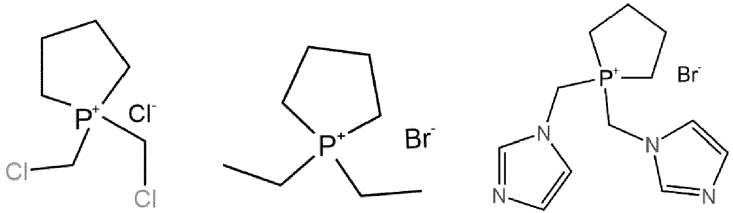

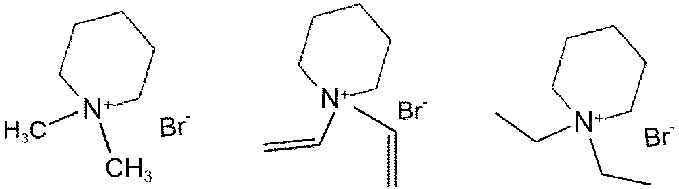

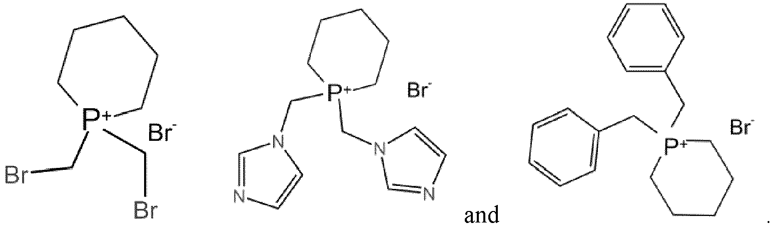

4. The use of claim 1, wherein the catalyst further comprises at least one compound of formula (2): 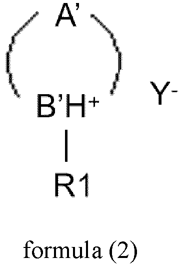 wherein R1 is selected from an alkyl group, an alkenyl group, a halohydrocarbyl group, an aromatic hydrocarbyl group, an imidazolyl group, or a heteroaromatic hydrocarbyl group; Y is selected from halogens; B' is selected from nitrogen, or phosphorus; A' is selected from any of the following formulae with a sign * representing a bonding position:

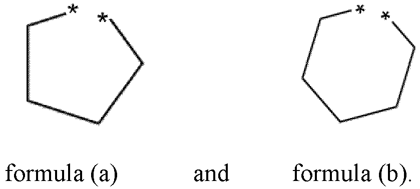

5. The use of claim 4, wherein R1 is selected from the alkyl group, the alkenyl group, or the halohydrocarbyl group, and has 1 to 15, preferably 1 to 10, carbon atoms; and/or, Y is selected from F, Cl, Br, or I, preferably, from Br, or Cl.

6. The use of claim 4, wherein the compound of formula (2) comprises at least one of:

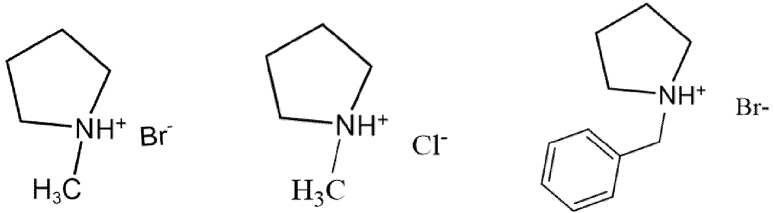

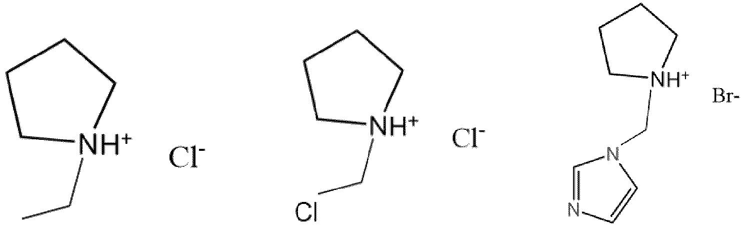

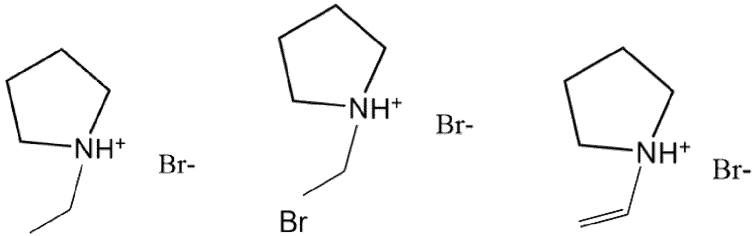

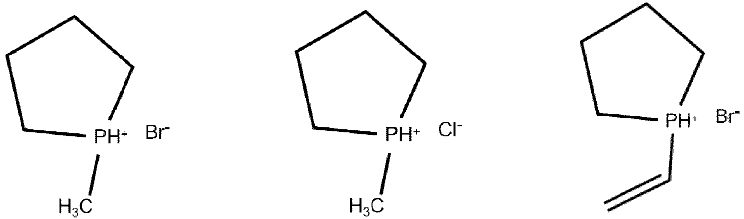

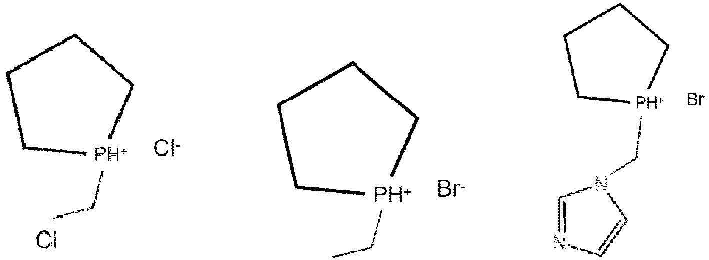

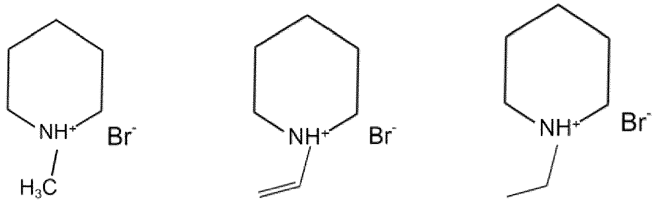

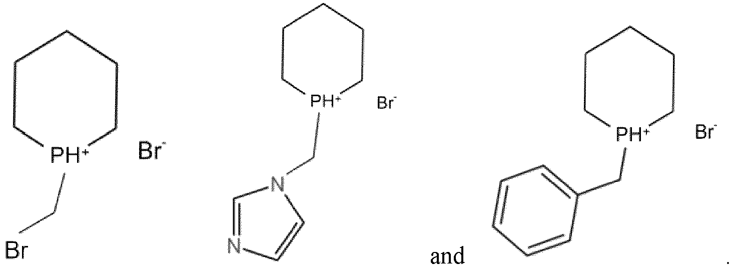

7. The use of claim 4, wherein a molar ratio of the compound of formula (1) to the compound of formula (2) is in a range of 100: (1 to 12).

8. The use of claim 1, wherein the epoxide compound is selected from at least one compound of formula (3): 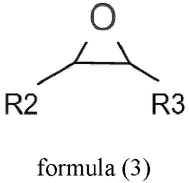 wherein when R2=H, R3 is selected from H, CH₃, CH₂Cl, C₂H₃, C₄H₉O, C₄H₉, C₆H₅, or C₈H₇O ; when R2≠H, the epoxide compound is cyclohexene oxide.

9. The use of any one of claims 1 to 8, wherein a molar ratio of the catalyst to the epoxide compound is in a range of (0.5 × 10⁻³ to 1.5 × 10⁻²):1.

10. The use of any one of claims 1 to 8, wherein the reaction is performed at a temperature of 100 to 200 °C, and/or under a pressure of 1 to 5 MPa.

11. A catalyst comprising at least one compound of formula (1) and at least one compound of formula (2): 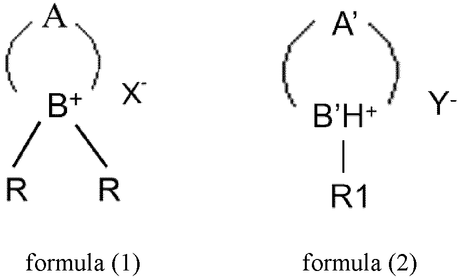
wherein R is selected from an alkyl group, an alkenyl group, a halohydrocarbyl group, an aromatic hydrocarbyl group, an imidazolyl group, or a heteroaromatic hydrocarbyl group; X is selected from halogens; B is selected from nitrogen, or phosphorus; and A is selected from any of the following formulae with a sign * representing a bonding position: 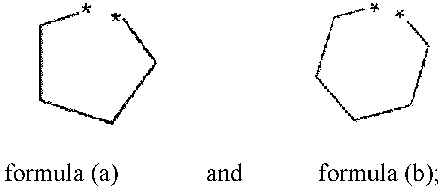
R1 is selected from an alkyl group, an alkenyl group, a halohydrocarbyl group, an aromatic hydrocarbyl group, an imidazolyl group, or a heteroaromatic hydrocarbyl group; Y is selected from halogens; B' is selected from nitrogen, or phosphorus; and A' is selected from any of the following formulae with a sign * representing a bonding position:

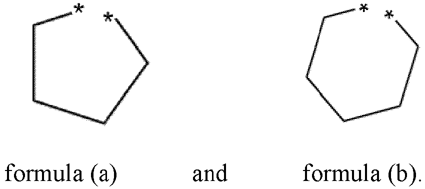

12. The catalyst of claim 11, wherein R is selected from the alkyl group, the alkenyl group, or the halohydrocarbyl group, and has 1 to 15, preferably 1 to 10, carbon atoms; and/or, X is selected from F, Cl, Br, or I, preferably from Br, or Cl.

13. The catalyst of claim 11, wherein the compound of formula (1) comprises at least one of:

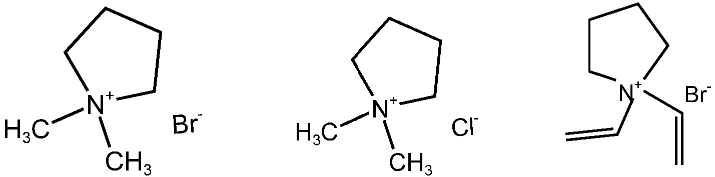

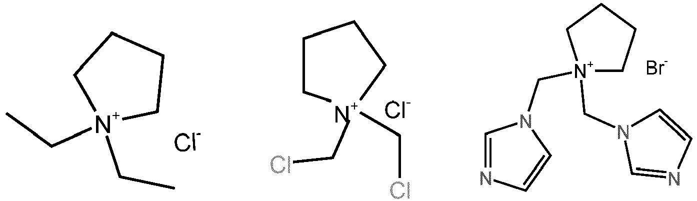

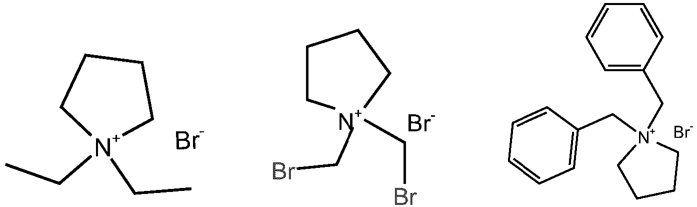

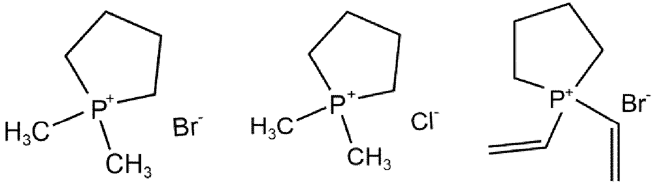

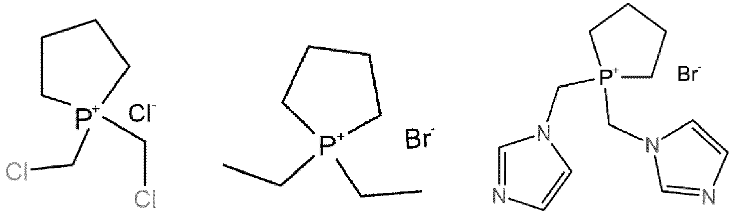

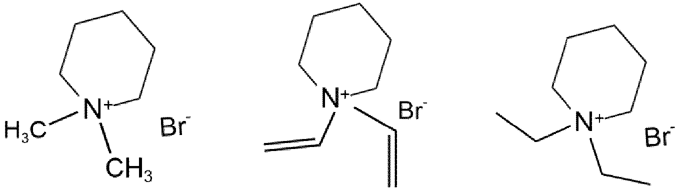

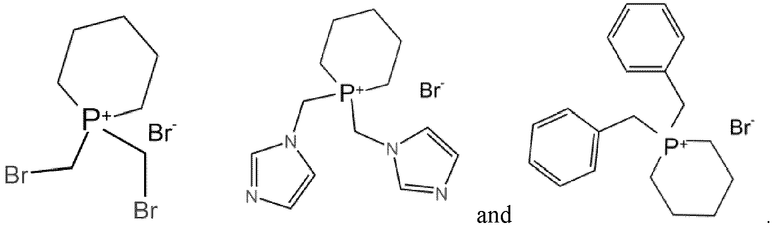

14. The catalyst of claim 11, wherein the compound of formula (2) comprises at least one of:
